# EUROPEAN PATENT APPLICATION

(11) **EP 4 523 549 A1**
(43) Date of publication of application: **19.03.2025**
(21) Application number: 23197216.7
(22) Date of filing: 13.09.2023
(51) Int. Cl.: A24B 13/00, A24B 15/30

(54) **ORAL PRODUCT COMPRISING THEOBROMINE**

(71) Applicant: Contraf-Nicotex-Tobacco GmbH, 74072 Heilbronn (DE)
(72) Inventor: Belz, Markus, 74074 Heilbronn (DE); Ellerichmann, Thomas, 74193 Schwaigern (DE); König, Thorsten, 95349 Thurnau (DE)
(74) Representative: Patentanwälte Ruff, Wilhelm, Beier, Dauster & Partner mbB

(57) **Abstract**

The invention relates to an oral product for delivery of at least one active ingredient comprising or consisting of
- a pouch defining a cavity and
- a composition comprising the at least one active ingredient and a carrier selected from the group consisting of, cocoa bean, in particular cocoa bean nibs or cocoa bean powder, coffee bean, cellulose, plant fibres, polysaccharides, oligosaccharides, polyols, and mixtures of at least two of the afore-said carriers,
wherein the composition is within the cavity of the pouch and the at least one active ingredient is theobromine.

The invention further relates to a method for producing an oral product.

## Description

### TECHNICAL FIELD AND PRIOR ART

The present invention relates to an oral product for delivery of at least one active ingredient comprising theobromine and to a method for producing an oral product.

### BACKGROUND OF THE INVENTION

Coffee consumption is a widespread habit all over the world, and consumption of caffeine, preferably contained in coffee, is the most consumed stimulant in the world.

Caffeine has a stimulant property on the human organism due to its relatively short-term and strong stimulating effect on the central nervous system, which, however, quickly wears off due to its relatively short half-life.

Therefore, some alternatives to the consumption of caffeine became widely available, for example the consumption of theobromine. Examples for natural sources of theobromine are cocoa beans of the cocoa tree (ca. 1-2.5% theobromine), the nut of the cola tree (Cola) (ca. 0.1 % theobromine), the leaves of the tea plant (ca. 0.05% theobromine) and the mate bush (ca. 0.1-0.2% theobromine).

As a stimulating substance, theobromine is often confused with caffeine. Overall, the effect of theobromine on the human organism is comparable to that of caffeine with less impact. The stimulating effect of theobromine is more permanent and also milder.

Because of the mild stimulating effect of theobromine in combination with low concentrations, the consumption of theobromine over its natural sources, e.g. cocoa or chocolate, is often not sufficient to feel a sufficient stimulating effect.

Despite the progress already made in the development of oral products containing active ingredients, in particular nicotine and caffeine, there remains further need to improve the delivery of theobromine as an active ingredient in the categories of user experience, in particular easy to be used, sensory attractiveness, sufficient stimulating effect and a fast relief of a craving.

The present invention addresses all of these points, as the inventive oral product allows a sufficient stimulating effect of theobromine, a comparatively fast release of theobromine and a subsequent faster uptake of theobromine by the mucosa directly into the blood stream, a superior sensory experience and an easy and low effort to produce the oral product. Furthermore, as another advantage of the oral product theobromine can be easily combined with any other active ingredients, in particular caffeine.

### OBJECT AND SOLUTION

In view of the foregoing, the object underlying the present invention is therefore to make available an oral product which properly addresses the above-mentioned need.

This object is accomplished by an oral product according to independent claim 1 and by a method according to claim 15. Preferred embodiments of the oral product are defined in the dependent claims 2 to 14. Further preferred embodiments of the invention are defined in the present description. The subject-matter and wording, respectively of all claims is hereby incorporated into the description by explicit reference.

According to a first aspect, the present invention relates to an oral product, in particular for delivery of at least one active ingredient, comprising a pouch defining a cavity and a composition comprising the at least one active ingredient and a carrier.

The carrier is selected from the group consisting of cocoa bean, in particular cocoa bean nibs or cocoa bean powder, coffee bean, cellulose, in particular microcrystalline cellulose, native cellulose or modified cellulose, plant fibres, in particular potato fibres, pea fibres, corn fibres, wheat fibres, apple fibres, coconut fibres, oat fibres or other plant fibres, polysaccharides, in particular native starches, resistant starches, modified starches or inulin, oligosaccharides, in particular maltodextrin or other oligosaccharides, polyols, in particular erythritol, maltitol, xylitol, mannitol or sorbitol and mixtures of at least two of the afore-said carriers.

The at least one active ingredient is theobromine.

Preferably, the oral product, in particular for delivery of an active ingredient, consists of a pouch defining a cavity and a composition comprising the at least one active ingredient and a carrier.

In other words, the active ingredient, preferably theobromine, is contained in the composition.

The oral product, preferably the composition, is capable of releasing the at least one active ingredient, preferably theobromine, without the need of chewing the oral product.

Preferably, the oral product, in particular the composition, is capable of releasing the active ingredient, in particular theobromine, by contact with mucosa, saliva, water and/or aqueous solutions.

The oral product may be at least partly, in particular only partly or preferably completely, biodegradable.

An advantage of a biodegradable oral product is a reduced risk of environmental pollution from the oral product, as it allows for rapid degradation in the environment and a reduction of plastic waste.

The oral product may be used for delivery of at least on active ingredient, in particular theobromine, and comprises theobromine.

Preferably, the oral product is wholly receivable in an oral cavity, in particular an oral cavity between gum and cheek.

Further preferred, the composition is a water-insoluble, preferably a partly water-insoluble, composition. An advantage of a water-insoluble, preferably partly water-insoluble, composition is, that most of the composition, in particular the water-insoluble components of the composition, preferably the carrier, remains in the pouch, whereas the at least on active ingredient, in particular theobromine, and/or other components added to the composition, are released from the composition, preferably by the saliva due to their (partial) water solubility.

Alternatively, the composition is a water-soluble composition.

The term "oral product" as used according to the present invention means a product which is intended to be taken into the mouth and preferably to be in contact with the oral mucosa while it remains in the mouth for a period of time but is not swallowed and preferably not chewed.

The term "layer" as used according to the present invention means a layer of a material, in particular a layer of a fabric material, which includes various fibre-based materials, including but not limited to fibres, yarns, filaments and threads.

The term "at least one layer" as used according to the present invention means one layer or a plurality of layers, i.e. two or more layers.

The term "biodegradable", as used according to the present invention, means an at least partial degradation or decomposition, i.e. chemical breakdown, of a material when being exposed to environmental influence, in particular rain, humidity and sunray.

The term "active ingredient" as used according to the present invention means a substance which in an organism has a specific action and/or evokes a specific response.

The term "at least one active ingredient" as used according to the present invention means one active ingredient or a plurality of active ingredients, i.e. two or more active ingredients.

The term "pouch" as used according to the present invention means a, preferably closed, layering, container or bag, providing a room or cavity, preferably an open room or cavity or a closed room or cavity, for storage in its inside, preferably between the layers of the pouch, which defines a room or cavity, preferably the room or cavity is open at 2 to 4 sides and/or covering its inside at 2 to 4 sides.

The term "cocoa nibs" as used according to the present invention means pieces of crushed cocoa beans. Cocoa nibs are preferably produced from cocoa beans, which are dried after harvesting, then fermented and cracked to produce small, dark bits.

The term "native cellulose" as used according to the present invention means lignocellulose, preferably plant dry matter. Lignocellulose forms the cell wall of lignified plants and serves as their structural framework. Lignocellulose is built by hemicelluloses and cellulose initially forming a scaffold into which lignin is subsequently incorporated during the process of lignification.

The term "modified cellulose or modified fibres" as used according to the present invention means a chemically modified cellulose. The chemical modifications are preferably made to the hydroxyl groups of the cellulose backbone.

The term "native starch" as used according to the present invention means pure forms of starch. They can be obtained from sources such as corn, wheat, potato, rice, cassava and tapioca. These long-chain carbohydrates are insoluble in cold water and swell to different degrees, depending on type and temperature.

The term "resistant starches" as used according to the present invention means starches that are resistant to digestion. This includes starches extracted from cereals such as oats, rice, corn or wheat, and tapioca, potato, green bananas, beans, and legumes.

The term "modified starches" as used according to the present invention are also called starch derivatives, and are starches that are modified, preferably by physical, enzymatical, or chemical treatment to change its properties.

The present invention is characterized in particular by the following advantages:
- The oral product allows a comparatively fast release of active ingredients, in particular theobromine, and subsequent a faster uptake of the active ingredient, in particular compared to the natural sources of the active ingredient, in particular theobromine.
- The oral product shows a high overall stability.
- A superior sensory experience can be achieved by the oral product, in particular due to the carrier, preferably cocoa nibs.
- Manufacturing effort and production costs are low due to the pouch structure.
- The oral product is suitable for the delivery of multiple active ingredients combined with theobromine.
- The consumer dosage options of the active ingredient, in particular theobromine, are better through the variable dimensions of the oral product.
- The oral product allows a comparatively fast release of additive compounds, in particular flavours.
- A chewing action is not needed for the release of the active ingredient, thereby a more sustained and consistent release of the active ingredient, preferably theobromine, can be achieved.

In an embodiment of the invention the carrier is cocoa bean, preferably cocoa bean nibs.

This is in particularly advantageous for the sensory experience of the consumer, since cocoa nibs have a typical slightly bitter, chocolate like taste, without having a high intake of calories, compared to known chocolate products.

In a further embodiment of the invention the carrier is present in the composition in amounts of 5 % by weight to 90 % by weight, in particular 20 % by weight to 80 % by weight, preferably 40 % by weight to 70 % by weight, based on the total weight of the composition.

In a further embodiment of the invention the carrier has a mean particle diameter of 10 µm to 5000 µm, in particular 100 µm to 500 µm, preferably 150 µm to 450 µm. The mean particle diameter mentioned in this paragraph is of particular advantage, as smaller mean particle diameters tend to leak out of the pouch and larger mean particle diameters do not provide enough surface area to carry the at least one active ingredient.

The above-described mean particle diameter of the carrier was found to be especially useful to obtain a homogenous distribution of the carrier in the composition.

Preferably the desired particle diameter of the carrier has been obtained by grinding, milling, in particular ball milling, homogenization, in particular high-pressure homogenization, micronization or a combination of at least two of the described methods.

The term "mean particle diameter" as used herein, means a particle or 50% of all particles of the carrier (D50) whose dimensions, in particular its diameter, preferably mean diameter, lies in a range from 10 µm to 5000 µm, in particular 100 µm to 500 µm, preferably 150 µm to 450 µm. In this context, the term "diameter" is to be understood in the case of a spherical particle to mean the diameter of the sphere, i.e. twice the radius of the sphere. In the case of a non-spherical particle, the term "diameter" is to be understood as the largest possible distance that two points along a circumference of the particle can take from each other.

More preferably, the carrier is in the form of particles, wherein 90% of all particles (D90) of the carrier are smaller than a particle diameter, in particular mean particle diameter, of 5 µm to 6000 µm, preferably of 2000 µm, more preferably of 1000 µm, even more preferred 500 µm.

Further preferred, the carrier is in the form of particles, wherein 50% of all particles (D50) of the carrier are smaller than a particle diameter, in particular mean particle diameter, of 10 µm to 5000 µm, in particular 100 µm to 500 µm, preferably 150 µm to 450 µm.

The mean particle diameter distribution is preferably obtained by sieve analysis. Sieve analysis is a method for determining the particle size distribution and is described in DIN 66165.

In a further embodiment of the invention the active ingredient, in particular theobromine is present in the composition in amounts of 1 % by weight to 50 % by weight, in particular 5 % by weight to 30 % by weight, preferably 10 % by weight to 20 % by weight, based on the total weight of the composition.

The afore-mentioned quantities of the active ingredient, preferably theobromine, are especially advantageous for a sustained release of the active ingredient and a stimulating effect on a body, in particular of theobromine.

The carrier, in particular cocoa beans, preferably cocoa bean nibs or cocoa bean powder, may comprise theobromine, in particular as natural source, in amounts of 0,01 % to 10%, preferably of 0,05% to 5%, more preferably of 0.5% to 3%, based on the total weight of the cocoa beans, preferably cocoa bean nibs or cocoa bean powder. In that case, the above-mentioned amount of theobromine is preferably to be understood as the amount of theobromine added to the composition, in particular added to the carrier, based on the total weight of the composition, preferably excluding an amount of theobromine contained in the carrier, or alternatively including an amount of theobromine contained in the carrier.

Alternatively, the cocoa beans, preferably cocoa bean nibs or cocoa bean powder, can be free of theobromine.

In a further embodiment of the invention the active ingredient is released from the composition or oral product during a period of 0,5 min to 60 min, in particular 1 min to 30 min, preferably 1 min to 10 min.

In a further embodiment of the invention the oral product, in particular the composition, comprises a pH regulating agent selected from the group consisting of carbonates, in particular sodium carbonate (Na2CO3), hydroxides, in particular sodium hydroxide (NaOH), acids, in particular citric acid, and salts thereof, fatty acids and salts thereof, buffer systems, and mixtures of at least two of the afore-said pH regulating agent.

The pH regulating agent may have a proportion of 0.1 % by weight to 10 % by weight, in particular 0.5% by weight to 5 % by weight, preferably 1 % by weight to 3 % by weight, based on the total weight of the composition.

The composition may have a pH of 2 to 10, in particular a pH of 4 to 9, preferably a pH of 5 to 8.

Alternatively, the oral product, in particular the composition, comprises no pH regulating agent, in particular none of the afore-mentioned pH regulating agents.

In a further embodiment of the invention the at least one active ingredient, in particular theobromine is in a free form, i.e. not ionic and/or bound form, in an ionic form, i.e. in the form of a salt, or in a bound, in particular complexed, form.

The active ingredient, in particular theobromine may be bound to a compound, in particular a polymer, preferably selected from the group consisting of mineralic polymers, natural polymers, synthetic polymers and mixtures of at least two of the afore-said carriers.

Alternatively, the active ingredient, in particular theobromine, may be bound to an ion-exchange resin, in particular selected from the group consisting of copolymers of divinylbenzene and methacrylic acid and mixtures of at least two of the afore-said ion-exchange resins.

Alternatively, the active ingredient, in particular theobromine, may form a complex, in particular with cyclodextrins.

Theobromine is an alkaloid from the methylxanthine group and can also be known under the name of 3,7-Dimethylxanthin and may be produced by chemical synthesis or by extraction from a natural product, especially by extraction from cocoa beans.

In a further embodiment of the invention the oral product, in particular the composition, comprises at least one, preferably further, in particular additional, active ingredient besides theobromine selected from the group consisting of amino acids, in particular tryptophane, theanine, tyrosine, or tryptophane derivates, in particular 5-HTP (5 Hydroxytryptophan), alkaloids, in particular nicotine, anatabine, caffeine, theobromine or theophylline, natural or synthetic cannabinoids, in particular cannabinol, cannabidivarin, cannabichromen, cannabidiol, cannabigerol, tetrahydrocannabivarin, Delta-8-tetrahydrocannabinol, Delta-9-tetrahydrocannabinol or Delta-x-tetrahydro cannabidiol, hormones, in particular melatonin, amino acids, in particular taurine or lysine, vitamins, minerals and mixtures of at least two of the afore-said active ingredients.

Preferably, at least one further, in particular additional, active ingredient besides theobromine is nicotine and/or caffeine.

Advantageously, the composition, enables the oral product for the delivery of multiple active ingredients other than or additional to theobromine.

Further, the oral product and/or composition may be free of tobacco or may contain or comprise tobacco.

In a further embodiment of the invention the oral product, in particular the composition, comprises at least one humectant selected from the group consisting of glycerol, propylene glycol, polydextrose, fructo-oligosaccharides, honey, molasses, and mixtures of at least two of the afore-said humectants.

The term "molasses" as used according to the present invention means a viscous dark brown sugar syrup that is preferably a by-product of sugar production from sugar cane, sugar beets and also from sugar millet.

The humectant may have a proportion of 0,01 % by weight to 50 % by weight, in particular 0,1 % by weight to 10 % by weight, preferably 1 % by weight to 7 % by weight, more preferably 1% by weight to 5% by weight, based on the total weight of the composition.

The humectant, in particular the above-mentioned proportions of the humectant, is advantageously improving the homogeneity of the composition and prevents separation of the components of the composition.

In a further embodiment of the invention the oral product, in particular the composition, comprises at least one compacting agent selected from the group consisting of carboxymethyl cellulose (CMC), polyvinylpyrrolidon, hydroclloids, in particular xanthan gum, gum arabica or guar gum, pectine, modified fibres and mixtures of at least two of the afore-said compacting agents.

The compacting agent may have a proportion of 0,01 % by weight to 50 % by weight, in particular 0,1 % by weight to 10 % by weight, preferably 1 % by weight to 5 % by weight, based on the total weight of the composition.

The compacting agent, in particular the above-mentioned proportions of the compacting agent, is advantageously improving the density of the composition and acting like an adhesive between the components of the composition.

In a further embodiment of the invention the oral product, in particular the composition, comprises at least one edible fatty molecule selected from the group consisting of lauric acid, myristic acid, palmitic acid, palmitate, palmitoleate, hydroxypalmitate, arachidic acid, oleic acid, stearic acid, sodium stearat, calcium stearate, magnesium stearate, hydroxyoctacosanyl hydroxystearate, oleate esters of long-chain, esters of fatty acids, fatty alcohols, esterified fatty diols, hydroxylated fatty acid, hydrogenated fatty acid, preferably saturated or partially saturated fatty acids, partially hydrogenated soybean, partially hydrogenated cottonseed oil, aliphatic alcohols, phospholipids, lecithin, phosphathydil cholin, triesters of fatty acids, coconut oil, hydrogenated coconut oil, cocoa butter, palm oil, fatty acid eutectics, mono and diglycerides, poloxamers, block-co-polymers of polyethylene glycol, polyesters and mixtures of at least two of the afore-said fatty molecules.

The edible fatty molecule may have a proportion of 0,01 % by weight to 50 % by weight, in particular 0,1 % by weight to 10 % by weight, preferably 1 % by weight to 5 % by weight, based on the total weight of the composition.

In a further embodiment of the invention the oral product, in particular the composition, comprises at least one additive compound, preferably selected from the group consisting of salts, in particular sodium chloride, flavour, spices, herbs, sugar, sugar substitute, sweetener, in particular high intensity sweetener, polyol, colour, preservative and mixtures of at least two of the afore-said additive compounds.

Alternatively, the oral product, in particular the composition, comprises no additive compounds, in particular none of the afore-mentioned additive compounds.

The flavour may have a proportion of 0,01 % by weight to 50 % by weight, in particular 0,1 % by weight to 10 % by weight, preferably 1 % by weight to 5 % by weight, based on the total weight of the composition.

The sugar substitute may be selected from the group consisting of polyols, in particular erythritol, xylitol, mannitol, sorbitol, isomalt, Lactitol, maltitol and mixtures of at least two of the afore-said polyols or bulk sweeteners selected from the group consisting of maltodextrin, allulose and mixtures of at least two of the afore-said bulk sweeteners.

The sugar substitute may have a proportion of 0,01 % by weight to 50 % by weight, in particular 0,1 % by weight to 10 % by weight, preferably 1 % by weight to 8 % by weight, more preferably 1 % by weight to 5 %, based on the total weight of the composition.

The sweetener, in particular high intensity sweetener, may be selected from the group consisting of sucralose, aspartame, stevia extract, acesulfame-K, neotame, thaumatin, neohesperidin DC, cyclamate, monk fruit, advantame, saccharin, alitame and mixtures of at least two of the afore-said sweeteners.

The sweetener, in particular high intensity sweetener, may have a proportion of 0,0001 % by weight to 10 % by weight, in particular 0,01 % by weight to 5 % by weight, preferably 0.1 % by weight to 2 % by weight, based on the total weight of the composition.

The colour may be selected from the group consisting of approved food colours and mixtures of at least two approved food colours.

The preservative may be selected from the group consisting of approved food preservatives and mixtures of at least two approved food preservatives.

The afore-mentioned quantities of the additives are especially advantageous for a sustained and consistent release of the additive compounds in particular of the additive compounds functioning as technical aids, for example pH regulators, and the additive compounds functioning as sensorial aids, for example flavours and sweeteners, from the oral product, in particular from the composition.

Advantageously, the composition preferably enables the oral product for the delivery of multiple additive compounds.

In a further embodiment of the invention the oral product, in particular the composition, comprises water, in particular present in an amount of 0,5 % by weight to 50 % by weight, in particular 2 % by weight to 40 % by weight, preferably 5 % by weight to 30 % by weight, based on the total weight of the composition.

Alternatively, the oral product, in particular the composition, is free of water. Preferably, the oral product, in particular the composition, is substantially free of water.

The term "substantially free of water" as used according to the present invention means less than 10 % by weight, in particular less than 1 % to 5 % by weight, water.

Preferably, the oral product, in particular the composition, may be free of water, in particular substantially free of water, after the oral product, in particular the composition, is dried.

In a further embodiment of the invention the pouch is in the form of at least one layer, preferably textile layer, in particular in the form of at least one nonwoven fabric layer, at least one felt fabric layer, at least one knitted fabric layer or at least one woven fabric layer.

Preferably, the pouch is in the form of lipophobic pouch material, in particular lipophobic layer. This is especially advantageous when a carrier comprising fat, especially cocoa beans, is used.

Preferably, the layer is in the form of at least one nonwoven fabric.

Alternatively, the layer is in the form of at least one non-textile layer.

Preferably the at least one layer, in particular at least one textile layer, is in the form of at least one nonwoven fabric, at least one felt fabric, at least one knitted fabric, at least one woven fabric or combinations of at least two of the afore-said fabrics.

Preferably, the at least one layer is in the form of at least one nonwoven fabric.

In a further embodiment of the invention the pouch, in particular at least one textile layer, comprises or consists of water-insoluble fibres, in particular water-insoluble natural fibres, preferably water-insoluble plant fibres and/or water-insoluble animal fibres, and/or synthetic fibres or combinations of at least two of the afore-said fibres.

The natural fibres are preferably selected from the group consisting of cotton fabric, silk fabric, linen fabric, wool fabric, paper fabric and combinations of at least two of the afore-said natural fibres.

The synthetic fibres are preferably selected from the group consisting of lycra fabric, viscose fabric, nylon fabric, polyester fabric, rayon fabric, polyethylenterephthalat (PET) fabric and combinations of at least two of the afore-said synthetic fibres.

The non-textile layer is preferably selected from the group consisting of plastic layers, latex layers, foam layers rubber layers and combinations of at least two of the afore-said non-textile layers.

In other words, it can be preferable according to the invention for the oral product to be a single layer, preferably textile layer, oral product, preferably pouch, in particular comprising the inventive composition.

The pouch may have a proportion of 1 % by weight to 99.999 % by weight, in particular 25 % by weight to 50 % by weight, preferably 25 % by weight to 35 % by weight, or alternatively 1 % by weight to 10 % by weight, in particular 1 % by weight to 5 % by weight, based on the total weight of the oral product.

The pouch, preferably the at least one layer may have a thickness of 0.1 mm to 5 mm, in particular of 0.75 mm to 2 mm, preferably of 0.9 mm to 1.1 mm.

The afore-mentioned thickness of the at least one layer is especially advantageous for a sustained and consistent release of the active ingredient, in particular theobromine.

Preferably, the at least one layer and the composition may be equal or different in terms of thickness.

The oral product comprising or consisting of the at least one layer and the composition comprising the active ingredient, in particular theobromine, may have a thickness of 0,6 mm to 15 mm, in particular of 1 mm to 8 mm, preferably of 2 mm to 5 mm.

Alternatively, the pouch is in the form of a plurality of layers, in particular 2 to 5 layers, wherein the layers are arranged one above the other.

In other words, it can be preferable according to the invention for the oral product to be a multilayer oral product, preferably a multilayer pouch, in particular comprising the inventive composition.

Preferably, the at least one layer comprises or consists of water-insoluble fibres, in particular water-insoluble natural fibres, preferably water-insoluble plant fibres and/or water-insoluble animal fibres, and/or synthetic fibres, more preferably selected from the group consisting of cotton fabric, silk fabric, linen fabric, wool fabric, lycra fabric, viscose fabric, nylon fabric, polyester fabric, rayon fabric, polyethylenterephthalat (PET) fabric and combinations of at least two of the afore-said fibres.

In a further embodiment of the invention the pouch is closed, in particular mechanically closed, preferably by means of heat sealing, stitching, embossing (imprinting) or needle felting.

The term "heat sealing" as used according to the present invention means to seal products, using heat. Preferably an adhesive, in particular a polymer adhesive, is used for the heat sealing.

The term "needle felting" as used according to the present invention means a mechanical connection of layers which is achieved when a needle is stabbed into layers, preferably arranged one above the other, multiple times. This process helps to entangle the material of the layers on itself leading to layers which are sticking together. The layers become firmly entangled together the more the needle is stabbed into it making the connection denser and firmer.

The term "stitching", also known as sewing, as used according to the present invention means a mechanical connection of layers, which is achieved when a needle connected with at least one thread is used to stab into layers, preferably arranged one above the other, multiple times, while the layers are connected by the help of the at least one thread.

The term "embossing" or "imprinting" as used according to the present invention means a mechanical connection of layers which is achieved when a stamp is put onto layers, preferably arranged one above the other, at least one time, preferably with a certain force and/or heat, to connect the layers.

The closing, in particular mechanical connection, of the pouch, preferably layers of the pouch, ensures that the inventive oral product comprising the inventive composition, stays intact and does not dissolve and/or separate during use.

Due to the closing, in particular a mechanical connection, of the layers by stitching, embossing or needle felting the manufacturing effort and production costs are relatively low, compared to other means of mechanically connecting layers, in particular by using chemical adhesives.

Advantageously, the oral product is easily adjustable in terms of its form or shape due to the layer structure, thereby allowing the release of the active ingredient, in particular theobromine and/or additive compounds to be easily adjustable.

Preferably, the at least one layer, forms a wall or is part of a wall of the pouch.

The composition may also be part of the wall of the pouch and/or be contained in a cavity surrounded by the wall of the pouch.

Preferably, at least two layers, in particular two layers, form the walls of the pouch, and the composition is surrounded by the at least two walls of the pouch, forming a cavity.

Alternatively, at least two layers, in particular two layers, form the walls of the pouch, and the composition is part of the wall of the pouch.

Preferably, at least two layers, in particular two layers, form the walls of the pouch, and at least one of the at least two layers is coated (layered) and/or penetrated by the composition.

Advantageously the at least one layer of the oral product protects the user from coming into direct contact with the composition and in particular the active ingredient, that may be absorbed through the skin before the intended use as an oral product in the mouth.

The afore mentioned forms of the oral product have the advantage to exhaustively release the composition, the active ingredient and/or the additives, preferably after 1 min to 10 min, so that the oral product, preferably the pouch, can be removed from the mouth in adequate time.

Alternatively, the oral product instead of being a pouch can be a sucking article such as bonbon, or a lozenge.

In a further embodiment of the invention the pouch, preferably the cavity of the pouch, is filled, coated, layered and/or penetrated by the composition.

In other words, it can be preferable according to the invention for the composition to be in the form of at least one layer of coating or layering on or in the cavity of the pouch.

Preferably, at least one outer layer of the pouch, in particular only one outer layer of the pouch, of the plurality of layers of the pouch is filled, coated, layered and/or penetrated by the composition.

This is in particularly advantageous since the pouch filled with, or coated or layered by, the composition and the composition, in particular composition layer, itself are wetted in the mouth at the same time due to contact with the saliva. Subsequently the composition, in particular water-soluble or partly water-soluble, composition, preferably dissolves and a fast release of the active ingredient, in particular theobromine, and a subsequent fast uptake through the oral mucosa can be achieved. A need for a chewing action to release the active ingredient from the oral product, which may lead to an unbalanced release of the active ingredient, in particular theobromine, is not given.

A further advantage of the pouch being filled with, or coated or layered by, the composition is, that the oral product allows a faster and more consistent release of the active ingredient, in particular theobromine, and additives and a subsequent faster and more consistent uptake of the active ingredient, compared to known oral products, in particular compared to oral products that are in the need of chewing to release ingredients, in particular active ingredients.

Preferably, the composition being in the form of a layer of coating or layering may have a layer thickness of 0,1 mm to 2,5 mm, in particular of 0,5 mm to 1,5 mm, preferably of 0,9 mm to 1,1 mm.

Further, the composition may have a proportion of 0.001 % by weight to 80 % by weight, in particular 1 % by weight to 70 % by weight, preferably 10 % by weight to 60 % by weight, more preferably 50 % by weight to 60 % by weight, based on the total weight of the oral product.

In a further embodiment of the invention the composition penetrates the at least one layer of the pouch. In other words the at least one layer of the pouch is penetrated by the composition.

Alternatively, the at least one layer of the pouch is coated or layered by the composition and/or the composition penetrates the at least one layer of the pouch and/or the pouch is filled with the composition.

Preferably, when penetrating the at least one layer of the pouch, the composition forms an inner composition layer within the pouch material. In other words, it can be preferable according to the invention for the composition to be in the form of an inner layer within the pouch material.

This is in particular advantageous since the layer penetrated by the composition is wetted in the mouth due to contact with the saliva. Only after the layer is wetted the inner composition layer, in particular water-soluble composition layer, is wetted and preferably dissolves and a consistent fast release of the active ingredient, in particular theobromine, from the layer and uptake through the oral mucosa and/or the gastro-intestinal system can be achieved. Therefore, there is no need for a chewing action to release the active ingredient from the oral product, which may lead to an unbalanced release of the active ingredient, in particular theobromine.

A further advantage of the pouch being penetrated by the composition is, that the oral product allows a faster and more consistent release of the active ingredient, in particular theobromine, and a subsequent faster and more consistent body uptake of the active ingredient, compared to known oral products, in particular compared to oral products that are in the need of chewing to release ingredients, in particular active ingredients.

In a further embodiment of the invention the composition is in the form of a hydrogel.

Preferably, the composition comprises a hydrogel forming component selected from the group consisting of carrageen, in particular kappa carrageen, agar-agar, alginates, polyvinyl pyrrolidone, gelatine, polyvinyl acetate, cellulose derivates, in particular carboxy methylcellulose (CMC) or hydroxypropyl methylcellulose (HPMC), and mixtures of at least two of the afore-said hydrogel forming components.

The hydrogel forming component may have a proportion of 0,01 % by weight to 50 % by weight, in particular 0,1 % by weight to 10 % by weight, preferably 0,1 % by weight to 3 % by weight, based on the total weight of the composition.

Preferably, the composition is in the form of a hydrogel before the oral product, in particular the composition, is dried.

The term "hydrogel" as used according to the present invention means a water containing polymer network which comprises a hydrogel forming component, preferably a long chain polysaccharide or protein.

According to a second aspect the invention relates to a method for preparing an oral product according to the first aspect comprising, in particular sequential in time, the steps of
a) providing a pouch,
b) providing a carrier,
c) preparing a composition by adding theobromine to the provided carrier,
d) filling, coating, layering and/or penetrating the provided pouch with the prepared composition, and
e) closing the pouch.
   wherein step c) is performed by
   - mixing the carrier with theobromine and/or
   - granulating the carrier in a dry granulation process, in particular by roller compaction, a wet granulation process, or a fluidized bed technology process and subsequently adding theobromine.

In step b) the carrier may be selected from the group consisting of cocoa bean, in particular cocoa bean nibs or cocoa bean powder, coffee bean, cellulose, in particular microcrystalline cellulose, native cellulose or modified cellulose, plant fibres, in particular potato fibres, pea fibres, corn fibres, wheat fibres, apple fibres, coconut fibres, oat fibres or other plant fibres, polysaccharides, in particular native starches, resistant starches, modified starches or inulin, oligosaccharides, in particular maltodextrin or other oligosaccharides, polyols, in particular erythritol, maltitol, xylitol, mannitol or sorbitol.

Preferably, step c) is performed at a temperature of 10 °C to 30°C, preferably of 15°C to 20°C.

Preferably, in step c) further components can be added to prepare the composition, preferably pH regulating agents, sugar substitutes, sweeteners, humectants and/or other additives, especially mentioned in the first aspect of the invention.

With respect to further features and advantages of the method, reference is made in its entirety to the features and advantages described in terms of the oral product according to the first aspect of the invention. The features and advantages described under the first aspect of the invention do apply, mutatis mutandis, with respect to the method according to the second aspect of the invention.

Further features and advantages of the invention will become clear from the following examples in conjunction with the subject-matter of the dependent claim. The individual features can be realized either singularly or severally in combination in one embodiment of the invention. The preferred embodiments merely serve for illustration and better understanding of the invention and are not to be understood as in any way limiting the invention.

### EXAMPLES

### Preparation of the inventive oral product

Cocoa nibs are milled taking into account a processing temperature, preferably of 10 °C to 30°C, more preferably of 15°C to 20°C, to prevent the cocoa fat from leaking.

The solid ingredients listed in table 1 are mixed with the milled cocoa nibs and in the next step the liquid ingredients mentioned in table 1 are added and mixed again.

This composition/granulate/mixture (also called pouch filler) is filled into the desired pouch material using a pouch machine.

For the pouch material a rather lipophobic pouch material is advantageous (because of the cocoa fat).

**Table 1: Composition of a 500mg oral product comprising theobromine**

| % | Raw material | CAS (if applicable) | Form |
|---|---|---|---|
| 7.8 | Erythritol (sugar substitute) | 69-65-8 | solid |
| 60.8 | Cocoa nibs, milled (carrier) | N/A | solid |
| 0.3 | Sucralose (sweetener) | 56038-13-2 | solid |
| 18.8 | Theobromine (active ingredient) | 83-67-0 | solid |
| 0.7 | Sodium chloride (additive) | N/A | solid |
| 6.6 | Glycerol (Humectant) | 56-81-5 | liquid |
| 5.0 | Flavour | N/A | liquid |

## Claims

1. An oral product for delivery of at least one active ingredient comprising or consisting of
- a pouch defining a cavity and
- a composition comprising the at least one active ingredient and a carrier selected from the group consisting of, cocoa bean, in particular cocoa bean nibs or cocoa bean powder, coffee bean, cellulose, plant fibres, polysaccharides, oligosaccharides, polyols, and mixtures of at least two of the afore-said carriers,
wherein the composition is within the cavity of the pouch and the at least one active ingredient is theobromine.

2. The oral product according to claim 1, **characterized in that** the carrier is present in amounts of 5 % by weight to 90 % by weight, in particular 20 % by weight to 80 % by weight, preferably 40 % by weight to 70 % by weight, based on the total weight of the composition.

3. The oral product according to claim 1 or 2, **characterized in that** the carrier has a mean particle size of 10 µm to 5000 µm, in particular 100 µm to 500 µm, preferably 150 µm to 450 µm.

4. The oral product according to any of the preceding claims, **characterized in that** the carrier is cocoa bean nibs.

5. The oral product according to any of the preceding claims, **characterized in that** theobromine is present in amounts of 1 % by weight to 50 % by weight, in particular 5 % by weight to 30 % by weight, preferably 10 % by weight to 20 % by weight, based on the total weight of the composition.

6. The oral product according to any of the preceding claims, **characterized in that** the composition comprises a pH regulating agent selected from the group consisting of carbonates, in particular sodium carbonate (Na2CO3), hydroxides, in particular sodium hydroxide (NaOH), acids, in particular citric acid, and salts thereof, fatty acids and salts thereof, and mixtures of at least two of the afore-said pH regulating agents.

7. The oral product according to any of the preceding claims, **characterized in that** the composition comprises at least one further active ingredient besides theobromine selected from the group consisting of amino acids, in particular tryptophane, nicotine, caffeine, taurine, cannabinoids, cannabinol, cannabidiol, cannabigerol, theophylline, tetrahydrocannabinol, melatonin, vitamins and mixtures of at least two of the afore-said active ingredients.

8. The oral product according to any of the preceding claims, **characterized in that** the composition comprises at least one humectant selected from the group consisting of glycerol, propylene glycol, polydextrose, fructooligosaccharide, honey, molasses and mixtures of at least two of the afore-said humectants.

9. The oral product according to any of the preceding claims, **characterized in that** the composition comprises at least one compacting agent selected from the group consisting of CMC, polyvinylpyrrolidon, hydrocolloids, in particular xanthan gum, gum arabica or guar gum, pectine and mixtures of at least two of the afore-said compacting agents.

10. The oral product according to any of the preceding claims, **characterized in that** the composition comprises at least one edible fatty molecule selected from the group consisting of lauric acid, myristic acid, palmitic acid, palmitate, palmitoleate, hydroxypalmitate, arachidic acid, oleic acid, stearic acid, sodium stearat, calcium stearate, magnesium stearate, hydroxyoctacosanyl hydroxystearate, oleate esters of long-chain, esters of fatty acids, fatty alcohols, esterified fatty diols, hydroxylated fatty acid, hydrogenated fatty acid, preferably saturated or partially saturated fatty acids, partially hydrogenated soybean, partially hydrogenated cottonseed oil, aliphatic alcohols, phospholipids, lecithin, phosphathydil cholin, triesters of fatty acids, coconut oil, hydrogenated coconut oil, cocoa butter; palm oil; fatty acid eutectics; mono and diglycerides, poloxamers, block-co-polymers of polyethylene glycol, polyesters and mixtures of at least two of the afore-said fatty molecules.

11. The oral product according to any of the preceding claims, **characterized in that** the oral product, in particular the composition, comprises at least one additive compound, preferably selected from the group consisting of salts, flavours, spices, herbs, sugar, sugar substitutes, sweeteners, colours, preservatives and mixtures of at least two of the afore-said additive compounds.

12. The oral product according to any of the preceding claims, **characterized in that** the composition comprises water, in particular present in an amount of 0,5 % by weight to 50 % by weight, in particular 2 % by weight to 40 % by weight, preferably 5 % by weight to 30 % by weight, based on the total weight of the composition.

13. The oral product according to any of the preceding claims, **characterized in that** the pouch is in the form of at least one non-textile layer or at least one textile layer in particular in the form of at least one nonwoven fabric layer, at least one felt fabric layer, at least one knitted fabric layer or at least one woven fabric layer.

14. The oral product according to any of the preceding claims, **characterized in that** the pouch is filled, coated, layered and/or penetrated by the composition.

15. A method for producing an oral product according any of the preceding claims, comprising the following steps
a) providing a pouch,
b) providing a carrier, selected from the group consisting of, cocoa bean, in particular cocoa bean nibs or cocoa bean powder, coffee bean, cellulose, plant fibres, polysaccharides, oligoschaccharides, polyols and mixtures of at least two of the afore-said carriers
c) preparing a composition by adding theobromine to the provided carrier,
d) filling, coating, layering and/or penetrating the provided pouch with the prepared composition, and
e) closing the pouch,
wherein step c) is performed by
- mixing the provided carrier with theobromine and/or
- granulating the provided carrier in a dry granulation process, in particular by roller compaction, a wet granulation process, or a fluidized bed technology process and subsequently adding theobromine.
